# EUROPEAN PATENT APPLICATION

(11) **EP 4 609 718 A1**
(43) Date of publication of application: **03.09.2025**
(21) Application number: 25160771.9
(22) Date of filing: 27.02.2025
(51) Int. Cl.: A23C 11/10, A23L 2/38, A23L 11/60, A23L 11/65, A23L 33/105, G01N 33/14, A23B 75/10, A23B 2/00, A23B 2/42, A23B 11/137, A23B 70/30, A23C 21/02, G01N 33/02, G01N 33/18

(54) **METHOD AND SYSTEM FOR PRODUCING A LIQUID FOOD PRODUCT**

(30) Priority: 29.02.2024 EP 24160684
(71) Applicant: Tetra Laval Holdings & Finance S.A., 1009 Pully (CH)
(72) Inventor: Bonneau, Paul, 92042 Paris La Défense Cedex (FR); Barr, Justin, 221 86 Lund (SE)
(74) Representative: Tetra Pak Patent Attorneys

(57) **Abstract**

The disclosure relates to a method (200) of producing a liquid food product (LP), the method (200) comprising
mixing (201) water (W) and a food material (F) to provide a mixture (M),
heat treating (202) the mixture (M) for deactivating harmful microorganisms,
evaporating (203) water from the mixture (M), to provide a stream of water vapor (WV), and a stream of liquid that forms the liquid food product (LP),
condensing (204) the water vapour (WV) to form a condensate (WC),
measuring (205) a dissolved organic carbon (DOC) content (C-r1) and/or a conductivity in at least one of the mixture (M), the condensate (WC) and the liquid food product (LP),
determining (206) a DOC content value (C-r2) and/or a conductivity value that represents the respective measured DOC content (C-r1) and conductivity, and
determining (207) whether the DOC content value (C-r2) and/or the conductivity value exceeds a respective threshold (ΔC-n) that is based on a respective nominal DOC content value (C-n) and a respective nominal conductivity value. The disclosure also relates to a system.

## Description

### Field of invention

The invention relates to a method of producing a liquid food product.

The invention also relates to a system for producing a liquid food product.

### Technical Background

Plant-based liquid food products are growing in popularity in recent years. To enhance the shelf-life of such products, it is commonly known to use different types of systems for providing heat treatment of liquid food in the food processing industry, also known as ultra-high temperature (UHT) systems. These systems are typically used for sterilizing the liquid food, as well as making the liquid food product ready for distribution efficiently and safely in relation to requirements related to the food processing industry.

Commonly, within all liquid food products that during production are to be mixed with water, the food product and water ratio is of importance. If the ratio deviates from the preferred values, the liquid food product may be diluted leading to deviant taste, colour or the like, or the product may be concentrated leading to a product which contains more food product than expected, and thereby the profit margin is reduced.

To control the ratio between food product and water in the liquid food product, the end product might be tested. If the test indicates that the product is too diluted or too concentrated, a large batch of the product may need to be discarded or recycled in the process. This implies large costs and ineffective production.

Unsatisfactory dilution may e.g., occur if the heat treatment device is malfunctioning or has incorrect settings.

Thus, there is still a need for a production method and system solution that fulfils the requirements related to the food processing industry, while at the same time ensuring good product quality, correct content ratio, and minimising product that go to waste or have to be processed again.

### Summary of invention

It is an object of the invention to provide a method and a system addressing at least some of the problems and fulfilling the need of a method that ensures good product quality.

This object has been achieved by a method of producing a liquid food product, the method comprising
mixing water and a food material to provide a mixture,
heat treating the mixture for deactivating harmful microorganisms,
evaporating water from the mixture, to provide a stream of water vapor, and a stream of liquid that forms the liquid food product,
condensing the water vapour to form a condensate,
measuring a dissolved organic carbon content and/or a conductivity in at least one of the mixture, the condensate and the liquid food product,
determining a DOC content value and/or a conductivity value that represents the respective measured DOC content and conductivity, respectively, and
determining whether the DOC content value and/or the conductivity value exceeds a respective threshold that is based on a respective nominal DOC content value and a respective nominal conductivity value.

This object has also been achieved by a system for producing a liquid food product, the system comprising
a mixing arrangement configured to mix water and a food material to provide a mixture,
a heat treatment arrangement configured to heat treat the mixture for deactivating harmful microorganisms,
a flash vessel or an evaporator configured to evaporate water from the mixture, to provide a stream of water vapor, and a stream of liquid that forms the liquid food product,
a condenser configured to condense the water vapour to form a condensate,
a control unit comprising software instructions for
measuring a DOC content and/or a conductivity in at least one of the mixture, the condensate, and the liquid food product,
determining a DOC content value and/or a conductivity value that represents the measured DOC content and/or the measured conductivity, and
determining whether the DOC content value and/or conductivity value exceeds a respective threshold that is based on a nominal DOC content value and the nominal condensate conductivity value.

The food material used when producing a liquid food product naturally contains carbon. Total organic carbon, TOC, is a measure of dissolved and particulate organic carbon in a sample. When measuring the carbon concentration in liquids, dissolved organic carbon, DOC, is commonly used.

By measuring the DOC content in the condensate and/or the liquid food product, it is possible to determine whether the liquid food product contains the desired content ratio, i.e., the desired ratio of water in relation to food product, and if a system cleaning process should be initiated or not. It has been realised that variations of DOC in the water condensate and/or in the liquid food can indicate that the content ratio in the liquid food product is off, i.e., the liquid food product is diluted or too concentrated, and an increase of DOC may also indicate that there is fouling in the production system and that the quality of the liquid food product will be negatively affected.

Increased levels of DOC may be caused by: increased evaporation rate, food material quality, and/or fouling in the system.

Decreased levels of DOC may be caused by decreased evaporation rate.

Alternatively, or additionally, the concentration and some aspects of the quality of the liquid food product, and/or any fouling of the system may be observed by measuring the conductivity in at least one of the mixture, the condensate, and the liquid food product, and preferably also in the supply water. Conductivity may be measured since the water condensate will contain ammonium ions. The ammonium ions may be present since some food materials, such as oat and soy, naturally includes certain amounts of ammonia or chemical compounds that naturally may give rise to ammonia. In the process of producing plant-based beverages, the food material is mixed with water, and enzymes may be added into the mixture to hydrolyse the plant-based food material to cut starch and form sugars. In the process of producing the plant-based beverages, substances within the food material may induce a chemical reaction with the enzymes, which are added, and/or present in the specific food material, when they are all mixed with water. In some cases, these chemical reactions result in the formation of ammonia. As an example, glutamine, which is present in oat and soybeans, may react with the enzymes added such that the glutamine and water reacts together with the enzymes such that the product is glutamate and ammonia. Whereby, ammonia, when dissolved in water, form ammonium hydroxide, consisting of the ammonium and hydroxide ions.

Increased levels of conductivity may be caused by: increased evaporation rate, food material quality, fermentation of the mixture and/or fouling in the system.

Decreased levels of conductivity may be caused by decreased evaporation rate and/or wrong dosage of enzymes in the holding stage.

Fermentation of the mixture may be caused by a food material that has started to ferment due to improper storage, the mixture is held within a storage tank for too long such that it starts to ferment, and/or by bacterial build-up in the production system.

As long as the DOC content and/or the conductivity in the liquid food product, and thus also in the water condensate, is close to a nominal value, the product quality is typically not affected. However, if the DOC content and/or the conductivity in the liquid food product, and consequently in the water condensate, increases or decreases such that the DOC content value exceeds or fall below a threshold value, the product quality is typically affected.

An advantage of the present method, and system, is that the control of the product's end quality is enhanced. Since the DOC content and/or the conductivity is/are monitored, necessary activities, to ensure high product quality, can be performed early, thus reducing the risk that the product is affected and thereby minimising the risk that the finished product must be discarded, or recycled in the process, which in turn ensures a cost- and resource-efficient production.

Another advantage is that the knowledge about the waste of the production process, i.e., the water condensate, is enhanced. By measuring and controlling the waste, suitable actions may be taken to ensure that the waste is handled properly and in accordance with existing governmental restrictions.

Heat treating the mixture is of importance to partly or fully sterilize the product and deactivating, or kill, harmful microorganisms such that the produced beverage is safe to consume. The heat treatment can be done by e.g., an indirect or direct heat treatment system, such as an UHT-system (ultra heat treatment or ultra high temperature system).

The determination of a DOC content value and/or a conductivity value and the determination of whether the DOC content value and/or conductivity value exceeds the respective threshold value may be performed by a control unit. The control unit may include a central processing unit, CPU, or any other control unit commonly known.

The control unit may receive information from at least one sensor which is configured to measure the DOC content in at least one of the mixture, the condensate, and the liquid food product. Alternatively, or additionally, the control unit may receive information from at least one sensor which is configured to measure the conductivity in at least one of the mixture, the condensate, the supply water and the liquid food product.

The control unit which is receiving information from at least one sensor configured to measure the DOC content and/or the conductivity in at least one of the mixture, the condensate, and the liquid food product may be one and the same, or the system may comprise several control units configured to receive and/or handle information from a respective sensor.

By measuring the DOC content in at least one of the mixture, the water condensate, and the liquid food product, and comparing the measured value, or a mean value of the measured values over time, with a nominal DOC content value, the DOC content value may be supervised. If the DOC content value reaches undesirable values, a control signal may be provided by the control unit allowing necessary actions to be made to prevent a faulty product. This is equally applicable when measuring conductivity or COD (chemical oxygen demand).

It is to be noted that the term "exceeds" refers to a value representing the DOC content value or the conductivity value, which is above or below a respective threshold, the respective threshold being e.g., a range of acceptable values based on the nominal DOC content value or the conductivity value. Thus, exceeding the threshold refers to values being greater than a maximum value of the acceptable range, or being less than a minimum value of the acceptable range.

The nominal DOC content value and the conductivity value may be a predetermined value that is associated with the type of food material to which the liquid food product belongs. As mentioned, food materials such as oats, and soy, naturally include carbon and ammonia, and thereby includes an expected amount of carbon and ammonia, and thus the nominal DOC content and the nominal conductivity can be predetermined based on the expected value.

Alternatively, or additionally, the nominal DOC content value may be determined by measuring the DOC content in at least one of the mixture, the condensate, and the liquid food product, during a first period of time, and determining a set of DOC content values that represent the DOC content measured during the first period of time, and thereafter setting the nominal DOC content value as a mean of the determined DOC content values. The first period of time may be a fixed, predetermined time. Alternatively, or as a complement, the first period of time may be adjusted such that a reasonable steady-state is obtained. This is equally applicable when determining the conductivity value.

It is to be noted that the DOC content and/or the conductivity may be measured by at least one sensor. The system may comprise one sensor but may alternatively comprise a plurality of sensors, e.g., a first, second, and third sensor.

The sensor may be configured to measure both the DOC content and the conductivity. Alternatively, the DOC content and the conductivity may be measured by a respective sensor.

The sensor configured to measure the DOC content may be a sensor capable of detecting DOC in a liquid, e.g., a sensor which utilizes fluorescence technology.

The sensor configured to measure the conductivity may be a sensor capable of detecting conductivity in a liquid, e.g., a sensor which utilizes electrode technology.

Alternatively, or additionally, the concentration of the liquid food product and/or fouling of the system may be observed by measuring the chemical oxygen demand, COD, in at least one of the mixture, the condensate, and the liquid food product. COD is the measure of the water's ability to consume oxygen during the decomposition of organic material in the water. In other words, it is the amount of oxygen needed to oxidize the organic material found in a quantity of water.

Increased levels of COD may be caused by: increased evaporation rate, food material quality, and/or fouling in the system.

Decreased levels of COD may be caused by decreased evaporation rate.

The sensor may be a sensor capable of detecting COD in a liquid, e.g., a sensor which utilizes UV optics.

Moreover, by measuring the conductivity in the supply water, it is possible to observe the conductivity from the start of the production process and thereby include such information when determining the conductivity value in e.g., the water condensate.

It may be noted that to determine if the liquid food product is diluted or too concentrated, measurements may be performed after the evaporation step, e.g., in the water condensate and/or the liquid food product.

It may be noted that to determine if the quality of the liquid food product is deteriorated due to fouling in the system, measurements may be performed before and/or after evaporation, e.g., in the mixture, the water condensate and/or the liquid food product.

It is to be noted that the heat treatment arrangement and evaporating arrangement may be an integrated system, such as a falling film evaporator, or a separate system, such as direct heating device and a flash vessel.

It is to be noted that the system may comprise any other commonly known heat treatment arrangement and/or evaporating arrangement.

In this context, the term "direct heating device" may be any device capable of increasing a temperature of a liquid or steam in an easy and efficient way, i.e., a device which is configured to heat the liquid or steam. By way of example, the direct heating device may be a steam injector or a steam infuser.

As readily appreciated by the person skilled in the art, the flash vessel should be interpreted as a vessel capable of performing flash evaporation (or partial evaporation). Flash evaporation (or partial evaporation) is the partial vapor that occurs when a saturated liquid stream, i.e., the stream, undergoes a reduction in pressure by passing through a throttling valve or other throttling device. Preferably, a part of the stream "flashes" into vapor in the flash vessel. Both the vapor and the residual of the stream are cooled to the saturation temperature of the stream at the reduced pressure.

During production, one purpose of the flash vessel is to expel water from the liquid food. Thus, when the liquid food is introduced via the direct heating device, the liquid food dilutes in the direct heating device. Having the flash vessel which is able to expel water from the liquid food, the flash vessel is advantageous as it is capable of restoring the liquid food as it was before being diluted in the direct heating device. Another purpose of the flash vessel during production is to quickly decrease the temperature of the liquid food. By way of example, the temperature of the liquid food is increased by the steam injector to approximately 140 °C and the temperature of the liquid food is decreased in the flash vessel to approximately 70-80 °C.

As readily appreciated by the person skilled in the art, the falling film evaporator should be interpreted as a device capable of evaporating fluid, such as water, from a product mixture. A falling film evaporator may advantageously be used when the liquid product is to be a condensate. An advantage of the falling film evaporator is that the product does not need to be diluted by e.g., steam, during the process of heat treatment.

It may be noted that the use of first, second, third, fourth, fifth, etc. are mainly to be seen as labels facilitating reading and that it does not necessarily mean that there need to be all the intervening numbers of portions present. It may e.g., be noted that it is contemplated to have a design where there is a first entity, a second entity, a third entity and a fifth entity, with a fourth entity being omitted. However, to facilitate reading, the numbering first, second, third, fourth, fifth, etc., is consistently used as labels for the respective entity, and in a sense based on an embodiment including all conceivable entities.

Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of said element, device, component, means, step, etc., unless explicitly stated otherwise. The steps of any method disclosed herein do not have to be performed in the exact order disclosed, unless explicitly stated.

### Brief description of the drawings

The invention will by way of example be described in more detail with reference to the appended schematic drawings, which shows a presently preferred embodiment of the invention.

Embodiments will now be described, by way of example, with reference to the accompanying schematic drawings, in which
Fig. 1A discloses a flowchart illustrating a Fig. 1A is a flowchart illustrating a first embodiment of a system for heat treatment of liquid food.
Fig. 1B disclose a flowchart illustrating a second embodiment of a system for heat treatment of liquid food.
Fig. 2 is a flowchart illustrating steps of a method for performing producing a liquid food product and measuring the DOC content value in the condensate.
Figs. 3 discloses different charts reflecting the measured DOC content in the water condensate over time.

### Detailed description of preferred embodiments

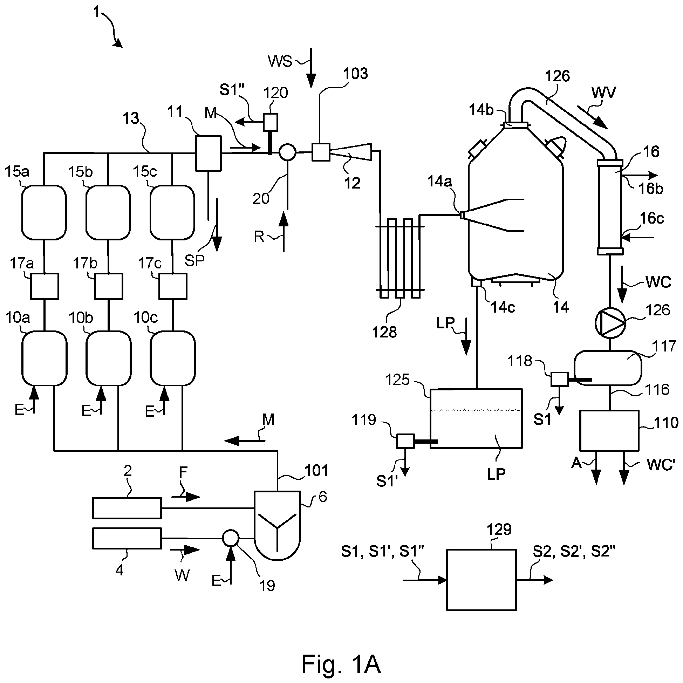

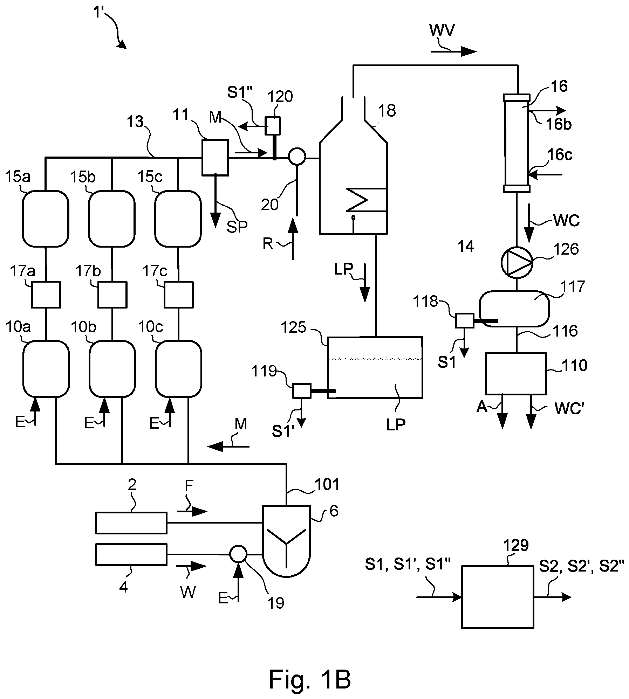

With reference to Fig. 1A and 1B, a flowchart of a system 1, 1' for heat treatment of food material mixture M is illustrated by way of example. The system 1, 1' is preferably an ultra-high temperature (UHT) system. The system 1, 1' is illustrated in a highly simplified manner in which only parts relevant for understanding how to perform measurements of the dissolved organic carbon (DOC) content C-r1 and/or the conductivity in at least one of the mixture M, the water condensate WC, and/or in the liquid food product LP. Hence, it is to be understood that the system 1,1' typically comprises further parts not shown or described in the following disclosure.

With reference to Fig. 1A, an exemplary system 1 for producing a plant-based liquid food product LP is illustrated, in which the system 1 generally uses plant-based food material F and water W to form a mixture M in a mixer 6. The plant-based food material F may be added to the mixer 6 from a container 2, such as a hopper, which may be adapted for conveying dry goods. The plant-based food material F may include any suitable type of food material F or combination of food material F for making the plant-based liquid food product LP. This includes, but is not limited to, one or more of oat, pea, soy, rice, beans or the like. The plant-based food material F may be in any suitable form when added to the mixer 6, such as a dry form, e.g., flour, flake, kernel, groat, seed, grains, pea, bean, nut, or the like, or any combination thereof. Additional ingredients may also be added to the mixer 6 to form the mixture M as may be desired to facilitate processing, taste, nutritional composition or other properties of the final liquid food product LP.

The water W may be added from a separate container or other source 4, such as a municipal water source. The water W is added to the mixer 6 as a liquid and may be heated prior to entering the mixer 6. Alternatively, or additionally, the mixture M may be heated in the mixer 6, such as with a heater in thermal communication with the mixer 6. The temperature of the water W or the mixture M in the mixer 6 may be in a range from 55 °C to 95 °C. The amount of water W added to the mixer 6 depends on the desired properties of the mixture M. For example, the mixture M may have a solids content in a range from e.g., 10% to 35%, or 16% to 20% by weight, or more, such that the amount of water W added to the mixer 6 may be in a range from 65% to 90% by weight, or less.

As seen in Figs. 1A-B, one or more containers, exemplified by three containers 10a-c, are located downstream of the mixer 6 on the line 101 and are configured to receive the mixture M and enabling the mixture M to be treated with enzymes E and/or with heat. That is, the mixture M may be treated either with enzymes, or with heat, or with both. Heat treatment may be done by supplying heat to the containers 10a-c, for example by using heating jackets that surround the respective container 10a-c. The enzymes E may, if used, hydrolyse the plant-based material in the mixture M to cut starch and create sugars. Following this process, the enzymes may be deactivated, either in the containers 10a-c, or in a separate deactivation container 17a-c which is located downstream of containers 10a-c.

Enzymes E may be added to product in the containers 10a-c, however, enzymes E could also be added directly at the mixer 6 via an enzyme inlet 19. In such a case, the containers 10a-c may be used for subsequent processing such as deactivation. The treatment with enzymes and/or heat may be performed according to known techniques commonly employed for the type of plant material that is used to form the liquid food product LP.

It is to be noted that there might be more, or less, than three containers 10a-c. Each container 10a-c may be fluidly connected with a respective deactivation container 17a-c, or each container 10a-c may be connected to one, or more, common deactivation containers 17a-c.

In the disclosed embodiment, a holding container 15a-c is located downstream of the enzyme deactivation container 17a-c, to hold the mixture M before it is further transported into the system 1, 1'.

There may be one holding container 15a-c for each deactivation container 17a-c as shown in Figs. 1A-1B, or each deactivation container 17a-c may be connected to one or more common holding containers 15a-c. The deactivation container 17a-c and the holding container 15a-c may be one and the same.

A separation unit 11 is located downstream of the mixer 6 and is configured to receive the mixture M and separate it into a solid portion SP and a liquid portion of the mixture, of which the solid portion SP may include fibrous material separated from the plant-based material F. The solids content in the liquid portion of the mixture M may be in a range from 10% to 30% by weight, for example, with a balance being the water from the mixture M.

The separation unit 11 may be a decanter or other suitable device. It is to be noted that the separation unit 11 may be optional, such as, when producing a hole-oat product, separation of the solid portion SP and the liquid portion is not desired and thus no separation unit 11 is present.

Downstream the separator, a first sensor 120 for measuring the DOC content C-r1 and/or the conductivity in the mixture M is located. The first sensor 120 provides the control unit 129 with a signal S1" comprising information about the measured DOC C-r1 and/or the measured conductivity. The appearance of an increased level of DOC content C-r1 at this stage of the process may e.g., be caused by fouling in the container 10a-c or the holding container 15a-c. The appearance of an increased level of conductivity at this stage of the process may e.g., be caused by fouling in the container 10a-c or the holding container 15a-c or by fermentation of the mixture M.

In the disclosed embodiment, the mixture M portion is subjected, as shown in Fig. 1A, to direct heating by a direct heating device 12 which is configured to receive the mixture M via a product inlet line 13, and a water steam WS via a steam inlet line 103. The direct heating device 12 is configured to create a stream comprising a mixture of the food material mixture M and the water steam WS, and consequently, the temperature of the mixture M is increased. The temperature of the stream may be at least 130°C when entering the flash vessel 14. Thus, the direct heating device 12 itself may be capable of heating the stream to at least 130°C. Additionally, or alternatively, the direct heating device 102 and the heating device 128 may together be capable of heating the stream to at least 130°C.

The stream, containing the mixture M and water steam WS, is fed to the flash vessel 14, via the heating device 128 which is configured to increase the temperature of the stream even further prior to entering the flash vessel 14. It is to be noted that an additional sensor may be provided in proximity to the heating device 128 to measure the conductivity in the stream and send a signal to the control unit 129. Due to the increased temperatures, substances in the mixture M may decompose which can give rise to increased ammonium levels, about an 10% increase, and thus, the conductivity in the stream may increase.

The stream enters the flash vessel 14 via a flash vessel inlet 14a. In the flash vessel 14, the stream pressure decreases, and consequently the temperature of the stream decreases, wherein the liquid food product LP is emptied from the flash vessel 14 at a bottom outlet 14c and the liquid food product LP enters an end product container 125. In the end product container 125, a second sensor 119 is located. The second sensor 119 measures the DOC content C-r1 and/or the conductivity in the produced liquid food product LP and provides a signal S1' to the control unit 129.

Liquid food product LP that may not adequately fulfil the quality requirements may be recycled into the process. The recycled product R is feed into the system through a recycle inlet line 20 such that the recycled product R can be heat treated once more to concentrate the product to a desired value or be diluted with more water and thereafter be heat treated once more.

A flow of water vapour WV is drawn from the flash vessel 14, via an upper outlet 14b of the flash vessel 14, to the condenser 16 where the water vapor WV is condensed to water condensate WC. The condenser 16 may be a tube heat exchanger in which a majority of the water vapor WV become condensate WC as well as the temperature of the water vapor WV may be decreased.

The condenser 16 comprises a fluid outlet 16b and a fluid inlet 16c.

Thereafter, the water condensate WC is drawn from the condenser 16 to a vacuum pump 126. A buffer container 117 is located downstream of the vacuum pump 126 so as to manage the flow from the vacuum pump 126. A third sensor 118 is located in the buffer container 117 to measure the DOC content C-r1 and/or the conductivity in the water condensate WC present in the buffer container 117. The third sensor 118 sends a signal S1 to the control unit 129.

The control unit 129 is configured to receive signals S1, S1', S" from one or more sensors 118, 119, 120 and to determine the DOC content value C-r2 and/or the conductivity value that represents the measured DOC content C-r1 and the measured conductivity, and determining whether the DOC content value C-r2 exceeds a threshold ΔC-n that is based on a nominal DOC content value C-n and/or determining whether the conductivity value exceeds a threshold that is based on a nominal conductivity value. If the DOC content value C-r2, at at least one measuring location, exceeds the threshold value ΔC-n, a control signal S2, S2', S2" is provided. The same reasoning may be applied relative to the determined conductivity value.

Downstream of the third sensor 118, along line 116, a water condensate handling arrangement 110 is located. The water condensate handling arrangement 110 may be configured to either to recover the water condensate WC' and e.g., recycle the water condensate WC', or be configured to remove i.e., drain, the water condensate WC'.

With reference to Fig. 1B, an alternative system 1' is shown. The system 1' as shown in Fig. 1B, have many similarities to the system 1 as shown in Fig. 1A. However, the alternative system 1' differs in that the heat-treating process and evaporation process is performed in an integrated system of an evaporator 18, such as a falling film evaporator. The evaporator 18 is located downstream of the separation unit 11 and is configured to receive and evaporate at least part of the mixture M to produce at least one evaporated portion in form of water vapor WV and at least one concentrated portion in form of the liquid food product LP. The water vapor WV is derived from the water W in the initial mixture M. Similar to the system 1, as shown in Fig. 1A, the system 1' comprises several sensors 118, 119, 120 which are arranged along the process to measure the DOC content C-r1 and/or the conductivity and send a signal S1, S1', S1" to the at least one control unit 129.

About 80% to 95%, or more, of the water in the mixture M may be evaporated into water vapour WV. The temperature of this evaporated water may be in a range from 70 °C to 90 °C. About 99% or more of the evaporated portion may be evaporated water, with a balance being impurities.

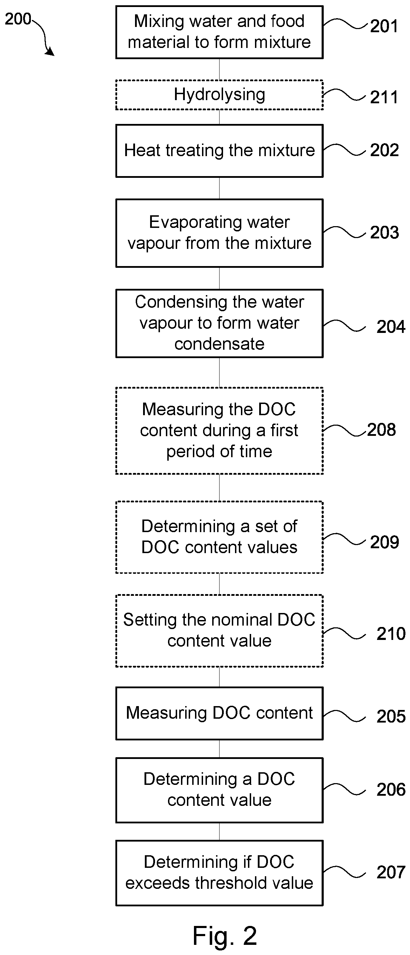

With reference to Fig. 2, a flowchart illustrating a method 200 for producing a liquid food product LP and measuring the DOC content C-r1 in at least one of the condensate WC, and the liquid food product LP, is shown. The method 200, as shown in Fig. 2, begins with a step of mixing 201 a at least a food material F and water W to form a mixture M. The mixture M may be prepared at an elevated temperature, such as by heating the water W or heating the mixture M.

The step 211 includes extracting nutrients from the food material F to the mixture M. The extracting of nutrients may be performed by treating the mixture M with enzymes E, which hydrolyse the food material F. The enzyme treatment may occur over a time span of 2, 4, 6, or even up to 15 hours. Generally, the amount of enzyme added may be less than 1% by weight of the total mixture M. Following enzyme treatment, the enzymes may be deactivated.

In step 202, the mixture M is be treated with heat, as appropriate in view of what type of plant-based liquid food product FP is prepared.

At step 202, the mixture M is heat treated by e.g., a direct steam injector 12 and the in step 203, the mixture M is evaporated by e.g., a flash vessel 14 such that the liquid food product LP and water vapor WV is formed. Alternatively, step 202 and step 203 are performed simultaneously in e.g., a falling film evaporator 18.

At step 204, the water vapor WV is drawn from the evaporator 18 or flash vessel 14 to the condenser 16 to condense the water vapor WV into water condensate WC.

At step 205, the DOC content C-r1 in at least one of the condensate WC, and the liquid food product LP is measured.

A DOC content value C-r2 is determined at step 206 and represents the measured DOC content C-r1.

It is determined in step 207 whether the DOC content value C-r2 exceeds a threshold ΔC-n that is based on a nominal DOC content value C-n.

The nominal DOC content value C-n, may, as will be described in further detail below, be based on a predetermined target value, or the method may, as shown in Fig. 2, comprise the steps of measuring 208, determining 209, and setting 210 a nominal content value C-n as a mean of the measured DOC content C-r1 during a first period of time Δt1. It is to be noted that the method as shown in Fig. 2, is equally applicable when it is desired to measure the conductivity and determine the conductivity value of the liquid food product LP.

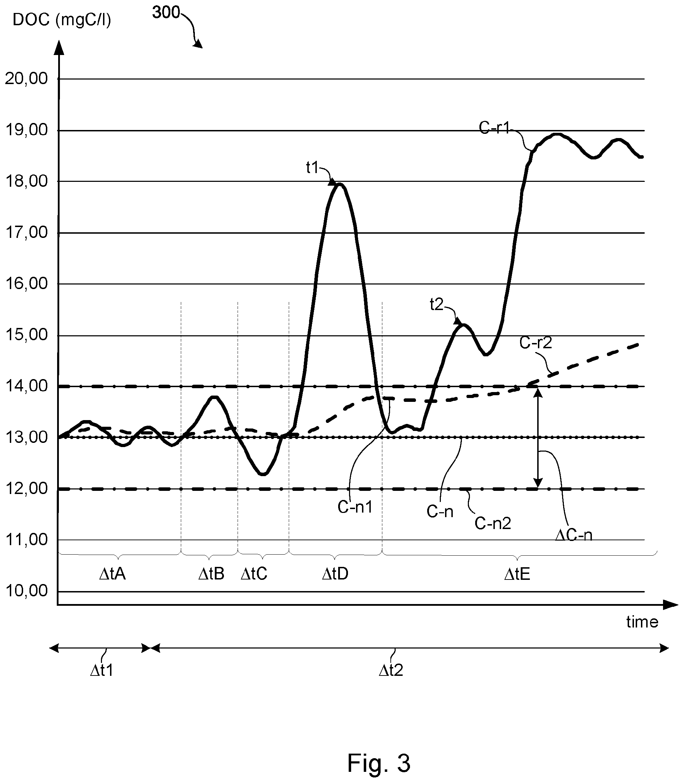

With reference to Fig. 3, an exemplified production process 300 of a soy-based liquid food product LP is shown as a graph. During the production process 300 the DOC content C-r1 is measured in at least the water condensate and/or the liquid food product LP. It has been realized that by measuring the DOC content value C-r2 at at least one of these stages of the production process, the control of the end product quality is enhanced, and knowledge about recurring events can be provided such that preventive actions may be taken to avoid, or at least reduce, deterioration of the finished product. A preventive action may be to plan more frequent sterilizations cycles. The DOC content varies over time for various reasons, as is described hereafter. Generally, the DOC variation is related to the variation of ammonia (NH₄) in the liquid food product LP.

It is to be noted that the exemplified production process 300, as shown in Fig.3, may be equally applicable to a production process of a liquid food product LP based on another type of plant-based material, e.g., oat.

The DOC content C-r1, measured in mg/l, and shown on the Y-axis, has been measured in the water condensate WC over a period of time, and the DOC content value C-r2 has been determined.

The production process 300, as shown in Fig. 3, extends over a time period of several hours.

The nominal DOC content value C-n may also be referred to as a reference value or a target value. The nominal DOC content value C-n may be based on: an assumption of the DOC content C-r1 associated with the specific food material F, a database specifying the DOC content C-r1 associated with the specific food material F, and/or by measuring the DOC content C-r1 in e.g., the water condensate WC, over a first period of time Δt1 to determine a steady state value of the DOC content C-r1. Wherein the set nominal DOC content value C-n is, during a second period of time Δt2, used for the determining 207 of whether the DOC content value C-r2 exceeds the threshold ΔC-n.

The nominal DOC value C-n in the water condensate WC during production of a plant-based beverage may be in the range of 10 to 15 mgC/I when the beverage is based on organic soy, and in the range of 12 to 20 mgC/I when based on oat.

It has been realised that the nominal DOC content value C-n generally is the same when the same food material F is used. Thus, if the food material F is changed, e.g., changing supplier of the soy based food material F, the nominal DOC content value C-n may change, and it may in such a case be advantageous to measure the DOC content C-r1 in e.g., the water condensate WC, over a first period of time Δt1 to determine a steady state value of the DOC content C-r1 of the new food material F.

The threshold ΔC-n may be an allowable variation of the DOC content value C-r2. The threshold ΔC-n has an upper threshold value C-n1 and a lower threshold value C-n2, wherein the threshold values C-n1, C-n2 comprises a range that is offset from the nominal DOC content value C-n. In Fig. 3, the threshold value C-n is set to be +/-1 mgC/l. It has been shown during process testing that the process itself may induce a variation of the measured DOC in the water condensate WC due to variation of e.g., flow. This observed process caused variation has not exceeded 1mgC/l. Thus, 1mgC/l could be a sufficient value to use as a threshold value. However, it is conceivable that greater threshold values are acceptable and it is also conceivable that smaller threshold values is considered acceptable. It is conceived that the threshold value C-n may be between 0,5 mgC/I and 5 mgC/l.

The DOC content value C-r2 represent a mean of measured DOC contents C-r1 during a period of time. By the term "mean" it intend to refer to one or more numbers or values that best represents a set of set of DOC content values C-r2. It may be the arithmetic mean, geometric mean, harmonic mean, generalized mean, weighted arithmetic, or any other suitable type of mean calculated by using known methods within the fields of mathematics and statistics.

The mean value of the measured DOC content C-r1 may be used to minimize the risk of causing false alarms. The term "false alarm" refers to a situation where the alarm is provided due to an isolated measurement exceeding the upper or lower threshold value C-n1, C-n2 which after a short time-period, e.g., a few minutes, returns, by itself, to an acceptable value. Irrespective of it is a measurement error or an actual isolated exceedance the quality of the liquid food product LP will not be noticeably affected, and thus no actions need to be taken. By instead using a mean value C-r2 of the measured DOC content C-r1, an alarm can be provided when the measured and determined DOC content value C-r2 has exceeded the upper or lower threshold value C-n1, C-n2 such that an action can be taken to prevent deterioration of the product quality. However, in some processes it might be favourable and desired to obtain an alarm on the instant measured DOC content C-r1 and not base the alarm set-up on a mean value of the measured DOC content C-r1. There may also be different thresholds used for instant alarms and for alarms based on mean values, respectively.

Fig. 3 illustrates different time intervals ΔtA-E which may occur during the production of a soy-based beverage. Note that these situations, which are to be described in further detail below, are equally applicable and realistic for a production process for a beverage based on another plant-base, such as oat.

Time interval ΔtA, as shown in Fig. 3, represent a normal production of a soy-based product. The DOC quantity is fluctuating around the nominal content value C-n which have been set to 13 mgC/l. The fluctuation may depend on the flow of the water condensate WC. The upper threshold value C-n1 is set to be 14 mg/l and the lower threshold value C-n2 is set to be 12 mg/l such that the allowable mean value C-r2 is set to be between 12 mg/l and 14 mg/l. As shown in time interval ΔtA, the DOC content value C-n2 is within the threshold ΔC-n, thus no alarm is activated. In a case where the system 1, 1' is set to provide an alarm based on the instant measured value C-r1, no alarm would be activated since the measured DOC content C-r1, during time interval ΔtA, also is within the set threshold ΔC-n.

During time interval ΔtB, as shown in Fig. 3, there is a peak of DOC content C-r1 in the water condensate WC. This small peak occurs for a short period of time and may be linked to a process adaptation, fouling in the system 1, 1', or increased evaporation. Increased evaporation can be linked to increased concentration of DOC content C-r1 in the water condensate WC since more water is extracted from the mixture M. Variations of measured DOC content C-r1 in the water condensate WC may also be caused by fouling in the production system 1, 1'. Fouling occurs when the mixture M is heated and substances in the mixture M e.g., proteins and fat molecules, stick to the hot surfaces of the system 1, 1'. One example could be that there occurs fouling in the holding containers 15a-c. The residue may come loose in blocks which will float around in the holding containers 15a-c, and at a certain point, a block of residue may follow the stream of the mixture M further into the system 1, 1' which will increase the measured DOC content C-r1. The measured DOC content C-r1 may be increased for a short period of time if the fouling is not that severe, however, as the system continues to run, more fouling is formed, and the DOC content C-r1 will increase.

"Process adaptation" refers to the stage after the machines in the system 1, 1' have been sterilised and/or where changes may have been made. A process adaptation may cause temporary unbalance in the production system 1, 1' which will, after some time, be stabilized.

As shown in time interval ΔtB, the measured DOC content C-n1 and the DOC content value C-n2 is within the threshold ΔC-n, thus no alarm is activated.

With reference to time interval ΔtC, as shown in Fig. 3, the DOC content level C-r1 in the water condensate WC have decreased. The small drop during time interval ΔtD may be linked to process adaptation, as discuses above, or decreased evaporation leading to less DOC concentration in the water condensate WC. A decreased DOC content value C-r2 caused by a decreased evaporation may imply that the product is diluted.

As shown in time interval ΔtC, the measured DOC value C-n1 and the DOC content value C-n2 is within the threshold ΔC-n, thus no alarm is activated.

As shown in Fig. 3, with reference to time interval ΔtD, a peak of the measured DOC content C-r1 is observed. The increased measured and determined DOC may be caused by the same aforementioned factors. If the alarm is set to be activated by the instant measured DOC value C-n1, the alarm would be activated due to the measured DOC content C-r1 has exceeded the upper threshold value C-n1. If the alarm is set to be activated by determined COD content value C-r2, an alarm would not be activated.

During the production process 300, as shown in Fig. 3, the alarm is set to be activated if the measured DOC value C-n1 would exceed the threshold ΔC-n two or three times in a short period of time, or if the determined DOC content value C-r2 exceeds the threshold ΔC-n. In case of an alarm, a water circulation could be initiated to hopefully remove fouling in the system 1,1'. If the desired result is not achieved by the action of cleaning the system 1, 1' by a water circulation process, an aseptic intermediate cleaning, AIC, could be initiated. AIC can be performed to prolong the production time between a full cleaning in place, CIP, process. When AIC is selected, the mixture M is displaced by sterile water before cleaning starts. During the AIC sequences, the holding tube is kept at the sterilization temperature, thus keeping the aseptic parts of the system sterile. The AIC can be performed either with lye only or lye and acid flush. As shown in time interval ΔtD in Fig.3, an AIC is initiated at t1 and the measured DOC content C-r1 is decreased.

With reference to time interval ΔtE, as shown in Fig. 3, a peak of measured DOC content C-r1 is observed, and the DOC content C-r1 does not decrease and seems to be stabilizing to a new value. It has been realized that when such a situation occurs, it is most often linked to that the fouling of the system 1,1' has increased to a level such that the production process may not be able to proceed. When the fouling reaches these high levels, the temperature in the system 1,1' drops. If the temperatures drop, there is a significant risk that the aseptic environment is lost, thus the liquid food product LP may not be safe to consume.

A first action may be to initiate an AIC process, which was done at t2, as seen in Fig. 3. However, the AIC may only be a temporary solution and the DOC values in the condensate would most probably only be decreased for a short period of time and thereafter once again increase. In such a case, a full cleaning in place, CIP, should be initiated. CIP can be defined as circulation of cleaning liquids through machines and other equipment in a cleaning circuit. The passage of the high-velocity flow of liquids over the equipment surfaces generates a mechanical scouring effect that dislodges dirt deposits. After the CIP has been performed, the production process may be started again, and the measured DOC content C-r1 should be back at the nominal DOC content value C-n.

Even though it is preferred that the system is designed in accordance with the disclosure in the detailed disclosure of preferred embodiments and the appended drawings, it should be noted that a specific preferred embodiment of a specific component does not necessarily have to be combined with a specific embodiment of another component. Thus, advantages associated with a specific embodiment, including one or more features, of a specific component may be accomplished even though the other component(s) is/are designed in accordance with the more general disclosure under the summary of the invention rather than being defined in accordance with the specific embodiment disclosed in the detailed description.

Additionally, variations to the disclosed embodiments can be understood and effected by the skilled person in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. A method (200) of producing a liquid food product (LP), the method (200) comprising
mixing (201) water (W) and a food material (F) to provide a mixture (M),
heat treating (202) the mixture (M) for deactivating harmful microorganisms,
evaporating (203) water from the mixture (M), to provide a stream of water vapor (WV), and a stream of liquid that forms the liquid food product (LP),
condensing (204) the water vapour (WV) to form a condensate (WC),
measuring (205) a dissolved organic carbon (DOC) content (C-r1) and/or a conductivity in at least one of the mixture (M), the condensate (WC) and the liquid food product (LP),
determining (206) a DOC content value (C-r2) and/or a conductivity value that represents the respective measured DOC content (C-r1) and conductivity, and
determining (207) whether the DOC content value (C-r2) and/or the conductivity value exceeds a respective threshold (ΔC-n) that is based on a respective nominal DOC content value (C-n) and a respective nominal conductivity value.

2. The method (200) according to claim 1, wherein respective nominal DOC content value (C-n) and respective nominal conductivity value is a respective predetermined value that is associated with the type of food product to which the liquid food product (LP) belongs.

3. The method (200) according to any preceding claim, further comprising
measuring (208), during a first period of time (Δt1), the DOC content (C-r1) and/or the conductivity in at least one of the mixture (M), the condensate (WC) and the liquid food product (LP),
determining (209) a set of DOC content values (C-r1) that represent the DOC content (C-r1) measured during the first period of time (Δt1) and/or determining a set of conductivity values that represent the conductivity measured during the first period of time (Δt1), and
setting (210) the nominal DOC content value (C-n) and/or the nominal conductivity value as a mean of the determined DOC content values (C-r1) and the determined conductivity value.

4. The method (200) according to claim 3, wherein the set nominal DOC content value (C-n) and/or the nominal conductivity value is/are, during a second period of time (Δt2) that is subsequent to the first period of time (Δt1), used for the determining (207) of whether the DOC content value (C-r2) and/or the conductivity value exceeds the respective threshold (ΔC-n).

5. The method (200) according to any preceding claim, wherein
the measuring (206) of the DOC content (C-r1) and/or the conductivity comprises measuring the DOC content (C-r1) and/or the conductivity during a period of time,
the determining (207) of the DOC content value (C-r2) and/or conductivity value comprises setting the DOC content value (C-r2) and/or the conductivity value as a mean of measured (206) DOC contents (C-r1) and/or the conductivity during said period of time.

6. The method (200) according to claim 5, further comprising
cleaning at least one component that is used for the producing of the liquid food product (LP), and, in response to said cleaning,
measuring the DOC content (C-r1) and/or the conductivity during a further period of time, and
setting the DOC content value (C-r2) and/or the conductivity value as a mean of the measured DOC contents (C-r1) and/or conductivity during said further period of time.

7. The method (200) according to any preceding claim, wherein the threshold (ΔC-n) of the DOC content value (C-r2) is a deviation from the nominal DOC content value (C-n) in the range from 0,5 to 5 mgC/l.

8. The method (200) according to any preceding claim, wherein the mixing (201) and the heat treating (202) is performed to produce a DOC content (C-r1) in the liquid food product (LP) that is
in the range from 10 to 20 mgC/l,
preferably in the range from 12 to 20 mgC/I when the food material (F) is oat, and
preferably in the range from 10 to 15 mgC/I when the food material (F) is soy.

9. The method (200) according to any of the preceding claims, wherein
the condensing (204) is performed by using a condenser (16), and
the measuring (205) of the DOC content (C-r1) and/or the conductivity in the condensate (WC) is performed by using at least one sensor (118, 119, 120) positioned in or downstream the condenser (16).

10. The method (200) according to claim 9, wherein the sensor (118, 119, 120) configured to measure the DOC content (C-r1) is a sensor which utilizes electrode technology.

11. The method (200) according to any preceding claim, wherein the measuring (205) of a DOC content (C-r1) and/or the conductivity comprises measuring the DOC content (C-r1) and/or the conductivity in the condensate (WC).

12. The method (200) according to any preceding claims, further comprising hydrolysing (211) the food material in the mixture (M) with at least one enzyme (E).

13. The method (200) according to claim 12, wherein the at least one enzyme (E) is one or both of amylase and protease.

14. The method (200) according to any preceding claims, wherein the food material (M) is a plant-based material, preferably oat, whey, rice, soy, or spelt.

15. A system (1, 1') for producing a liquid food product (LP), the system (1. 1') comprising
a mixing arrangement (6) configured to mix water (W) and a food material (F) to provide a mixture (M),
a heat treatment arrangement (12, 128, 18) configured to heat treat the mixture (M) for deactivating harmful microorganisms,
a flash vessel (14) or an evaporator (18) configured to evaporate water from the mixture (M), to provide a stream of water vapor (WV), and a stream of liquid that forms the liquid food product (LP),
a condenser (16) configured to condense the water vapour (WV) to form a condensate (WC),
a control unit (129) comprising software instructions for
measuring a DOC content (C-r1) and/or a conductivity in at least one of the mixture (M), the condensate (WC) and the liquid food product (LP),
determining a DOC content value (C-r2) and/or a conductivity value that represents the measured DOC content (C-r1) and/or the measured conductivity, and
determining whether the DOC content value (C-r2) and/or conductivity value exceeds a respective threshold (ΔC-n) that is based on a nominal DOC content value (C-n) and the nominal condensate conductivity value.
